# EUROPÄISCHE PATENTSCHRIFT

(11) **EP 3 582 841 B1**
(45) Veröffentlichungstag und Bekanntmachung des Hinweises auf die Patenterteilung: **29.06.2022**
(21) Anmeldenummer: 18712072.0
(22) Anmeldetag: 16.02.2018
(51) Int. Cl.: A61M 16/00, A61B 5/087, G01P 5/165, G01F 1/46, A61M 16/08, F04D 29/66, F04D 29/62, F04B 53/00

(54) **VORRICHTUNG ZUR BEATMUNG MIT EINEM GEBLÄSE-LAGERELEMENT UND LEITSTRUKTUR**
DEVICE FOR VENTILATION WITH A FAN BEARING ELEMENT AND CONDUCTING STRUCTURE
DISPOSITIF DE RESPIRATION AVEC UN ÉLÉMENT DE POSITION DE SOUFFLEUR ET STRUCTURE DE CONDUITE

(30) Priorität: 20.02.2017 DE 102017001558
(43) Veröffentlichungstag der Anmeldung: 25.12.2019
(73) Patentinhaber: WEINMANN Emergency Medical Technology GmbH + Co. KG, 22525 Hamburg (DE)
(72) Erfinder: DIEHL, Marcus, 22587 Hamburg (DE); HEIN, Stefan, 22529 Hamburg (DE); PULLA, Matthias, 22459 Hamburg (DE); HERRMANN, Frank, 25355 Barmstedt (DE)
(74) Vertreter: Klickow & Wetzel PartGmbB
(86) Internationale Anmeldenummer: PCT/DE2018/000043
(87) Internationale Veröffentlichungsnummer: WO 2018/149436

(56) Entgegenhaltungen:
- WO-A1-2013/114345
- WO-A1-2013/133889
- WO-A2-2007/024955
- DE-U1-202014 007 024
- US-B1- 7 975 688

## Beschreibung

Die Erfindung betrifft eine Vorrichtung zur Beatmung sowie eines nichttherapeutischen Verfahrens zum Betrieb der Vorrichtung zur Beatmung.

Insbesondere ist an eine Anwendung bei mobilen Notfallbeatmungsgeräten gedacht.

Im Falle unzureichender oder ausbleibender eigenständiger Atmung eines sauerstoffbedürftigen Lebewesens, muss die Atemarbeit als lebenserhaltende Maßnahme von einem Dritten entweder manuell, beispielsweise durch Mund-zu-Mund-Beatmung oder mithilfe von Beatmungsbeuteln, oder maschinell mit einem Beatmungsgerät durchgeführt werden, um den Gasaustausch in der Lunge sicherzustellen und so die Organe mit Sauerstoff zu versorgen sowie CO2 abzuatmen.

Eine sichere Methode ist nach dem Stand der Technik die maschinelle Beatmung, die in Form von Beatmungsgeräten zur medizintechnischen Ausstattung von Rettungskräften und Klinikpersonal gehört.

Moderne Beatmungsgeräte und -verfahren bieten mit einer Volumen- und/oder Drucckontrolle wichtige Funktionen zu einer effektiven und gleichzeitig schonenden Beatmung von Patienten. Ein zu hoher Druck kann das Lungengewebe schädigen, während ein zu kleines Beatmungsvolumen eine Unterversorgung mit Sauerstoff zur Folge hat. Auch die Frequenz der Beatmung ist mit bekannten Beatmungsgeräten präzise an internationale Richtlinien anpassbar, die eine effektive Beatmung, auch im Falle von Wiederbelebungsmaßnahmen, gewährleisten sollen.

Im Fall mobiler Beatmungsgeräte sind diese bei der Verwendung häufig Stößen, beispielsweise verursacht durch ein unsanftes Abstellen, Verladen oder durch Stürze, ausgesetzt. Die bei Stößen plötzlich auftretenden Kräfte können innerhalb eines Beatmungsgerätes auch an kritischen Orten auftreten, die durch diese plötzlich auftretenden Kräfte ggf. beschädigt werden können. Ein kritischer Ort ist beispielsweise das Gebläse, insbesondere das Lager und die Welle des Gebläses, die durch Querkräfte beschädigt werden können.

Weiterhin erzeugen die Gebläse von bekannten Vorrichtungen zur Beatmung häufig Körperschall und in Verbindung mit den Leitstrukturen der Vorrichtungen zur Beatmung turbulente Strömungen, die einerseits Schallemissionen und Vibrationen der Vorrichtung zur Beatmung zur Folge haben können und andererseits die häufig eingesetzte Volumenstrommessung des Atemgases negativ beeinflussen.

Gemäß dem Stand der Technik wird die Kühlluft von mobilen Beatmungsgeräten zum Abführen von Wärme nur während der Beatmung eines Patienten abgeblasen. Dies führt in einigen Fällen dazu, dass das Beatmungsgerät in einem eingeschalteten Zustand nicht ausreichend entwärmt wird und Schäden an Komponenten entstehen oder zumindest die Lebensdauer einzelner Komponenten verkürzt wird.

Aus der WO 2013/133 889 A1 ist bereits eine Vorrichtung zur Beatmung mit einem Lagerelement für ein Gebläse bekannt.

Ähnliche Vorrichtungen werden auch in der US 7 975 688 B1 sowie der WO 2007/024955 A2 beschrieben.

In der DE 20 2014 00 7024 U1 wird bereits eine Vorrichtung zur Beatmung beschrieben, die ein Gebläse mit einem Gebläsemotor aufweist und bei der der Gebläsemotor mit einem Lüfterrad versehen ist, und bei der zur Lagerung ein Lagerelement aus Silikon verwendet wird.

In der WO 2013/1143 45 A1 wird ebenfalls bereits eine Vorrichtung zur Beatmung beschrieben, die ein von einem Gebläsemotor angetriebenes Gebläse aufweist. Der Gebläsemotor wird von Lagerelementen gelagert. Die Lagerelemente können aus Silikon gefertigt sein und es ist eine Kühlung des Gebläsemotors unter Verwendung von Kühlluft vorgesehen.

Eine Aufgabe der Erfindung ist es, das Gebläse der Vorrichtung zur Beatmung unempfindlicher gegenüber Stößen zu machen.

Diese Aufgabe wird erfindungsgemäß durch die Merkmale von Patentanspruch 1 gelöst.

Eine weitere Aufgabe der Erfindung ist es, den störenden Einfluss des Gebläses auf die Volumenstrommessung der Vorrichtung zur Beatmung zu reduzieren.

Diese Aufgabe wird erfindungsgemäß ebenfalls durch die Merkmale von Patentanspruch 1 gelöst.

Eine weitere Aufgabe der Erfindung ist es, die Vorrichtung zur Beatmung in einem eingeschalteten Zustand permanent durch das Abblasen der Kühlluft zu entwärmen.

Diese Aufgabe wird erfindungsgemäß dadurch gelöst, dass das Gebläse auch in einem eingeschalteten Zustand der Vorrichtung zur Beatmung läuft, in dem keine Beatmung stattfindet.

Eine erfindungsgemäße Ausführungsform einer Vorrichtung zur Beatmung weist zumindest ein Gebläse auf, über das die Umgebungsluft über einen Filter in eine Gebläsekammer innerhalb des Gehäuses der Vorrichtung zur Beatmung ansaugbar ist. Das Gebläse ist als ein Gebläsemodul realisiert, das neben einem Lüfterrad zumindest einen Kühlkörper und einen Gebläsemotor aufweist. Das Gebläsemodul ist erfindungsgemäß mit mindestens einem Silikonteil zumindest an zwei Enden gelagert, sodass Emissionen und Immissionen, insbesondere im Sinne von Körperschall bzw. Vibrationen und Stößen, von bzw. auf das Gebläsemodul dämpfbar sind.

In einer vorteilhaften Ausführungsform der Erfindung ist das Gebläsemodul mithilfe von zwei Silikonteilen im Gehäuse gelagert, die als ein erstes Lagerelement und ein zweites Lagerelement ausgebildet sind.

Der das Gebläsemodul im Bereich des Kühlkörpers lagernde Bereich des Silikonteils bzw. das entsprechende Lagerelement weist mindestens eine Ausströmöffnung auf und ist weiterhin als eine Leitstruktur für Luft derart ausgebildet, dass die von dem Gebläse in die Gebläsekammer gedrückte Luft über den Kühlkörper zwangsleitbar ist, um einen effektiven Wärmeaustausch zu realisieren.

Die Kühlluft ist über eine Kühlblende aus dem Gehäuse abblasbar, die mindestens einen Auslass aufweist. Durch den Durchmesser des Auslasses bzw. der Auslässe in Verbindung mit dem Innendruck im Bereich der Kühlblende und der Rückstellkraft eines im Bereich des Auslasses angeordneten Rückschlagventils, ist der Volumenstrom der austretenden Kühlluft einstellbar.

In einer vorteilhaften Ausführungsform weist die erfindungsgemäße Vorrichtung zur Beatmung eine Kühlblende mit zwei als zylinderförmige Röhren ausgebildeten Auslässen auf, die einen Durchmesser von 1,5 mm bis 3,5 mm aufweisen und mit denen ein Kühlluftstrom zwischen 25 l/min und 75 l/min realisierbar ist.

In einer besonders vorteilhaften Ausführungsform der Erfindung weist die erfindungsgemäße Vorrichtung zur Beatmung eine Kühlblende mit zwei als zylinderförmige Röhren ausgebildeten Auslässen auf, die einen Durchmesser von 2 mm bis 3 mm aufweisen und mit denen ein Kühlluftstrom von etwa 50 l/min realisierbar ist.

In einer vorteilhaften Ausführungsform eines erfindungsgemäßen nichttherapeutischen Verfahrens zum Betrieb der Vorrichtung zur Beatmung strömt die Umgebungsluft über einen Hygienefilter in das Gerät ein. Von dort gelangt sie in den Ansaugbereich der Gebläsekammer, wird vom Gebläse angesaugt und über eine Öffnung in der Gebläsekappe in den Druckbereich der Gebläsekammer geblasen. Von hier strömt die Luft, zwangsgeführt durch das zumindest teilweise aus Silikon gefertigte Lagerelement des Gebläses, über die erfindungsgemäßen Kühlrippen des Kühlkörpers des Gebläses zum Kühlventil hin. Das Silikonteil erfüllt dabei eine doppelte Funktion: zum einen dient es als Dämpfer gegenüber Schock- und Vibration, bzw. Sturz des Geräts, zum anderen dient es als Zwangsführung für die Luft, die über die Kühlrippen des Kühlkörpers die Wärme des Gebläses abtransportiert.

In einer besonders vorteilhaften Ausführungsform des erfindungsgemäßen nichttherapeutischen Verfahrens zum Betrieb der Vorrichtung zur Beatmung wird die am Kühlkörper des Gebläses aufgenommene Wärme permanent nach außen abgeführt, auch bei fehlender Atemgasabgabe an einen Patienten.

Weiterhin ist in einer vorteilhaften Ausführungsform einer erfindungsgemäßen Vorrichtung zur Beatmung das die Leitstruktur für die Luft im Überdruckbereich realisierende Lagerelement derart ausgebildet, dass der Luftstrom beruhigt wird, sodass ein störender Einfluss auf eine im Bereich der erfindungsgemäßen Vorrichtung durchgeführte Volumenstrommessung vermindert wird. Dies ist erfindungsgemäß beispielsweise dadurch realisiert, dass das Lagerelement im Bereich des Kühlkörpers des Gebläses mehrere gleichartig ausgebildete Ausströmöffnungen aufweist, die den Luftstrom in Verbindung mit der Struktur des Kühlkörpers beruhigen.

Im Bereich des Kühlkörpers ist das Silikonteil bzw. das entsprechende Lagerelement in einer vorteilhaften Ausführungsform der Erfindung überzieherartig ausgebildet, sodass es ähnlich einer Hülse zumindest bereichsweise über den Kühlkörper des Gebläses stülpbar ist und diesen radial umlaufend umschließt.

Zusätzlich zur gedämpften Lagerung des Gebläses realisiert das Silikonteil bzw. realisieren die Lagerelemente in einer vorteilhaften Ausführungsform der Erfindung eine Abdichtung der verschiedenen Druckbereiche in der Umgebung des Gebläses voneinander. Die Abdichtung wird durch die Struktur des Silikonteils bzw. der Lagerelemente in Verbindung mit der Struktur des das Gebläse umgebenden Gehäuses beispielsweise derart realisiert, dass das Silikonteil bzw. das Lagerelement im Bereich der Ansaugöffnung des Gebläses diesen von einem ersten Überdruckbereich im Bereich des Kühlkörpers des Gebläses durch eine nut- und federartig ausgebildete Struktur abdichtet.

Verschiedene Ausführungsbeispiele und Ausgestaltungen der Erfindung sind in den nachfolgenden Figuren abgebildet. Es zeigen
- Figur 1:: eine perspektivische Darstellung einer erfindungsgemäßen Vorrichtung zur Beatmung,
- Figur 2:: einen horizontalen Schnitt einer erfindungsgemäßen Vorrichtung zur Beatmung, aufweisend eine stoßgedämpfte Gebläselagerung,
- Figur 3:: einen vertikalen Schnitt einer erfindungsgemäßen Vorrichtung zur Beatmung, aufweisend eine stoßgedämpfte Gebläselagerung,
- Figur 4:: eine perspektivische Ansicht eines erfindungsgemäßen Gebläsemoduls einer Vorrichtung zur Beatmung,
- Figur 5:: einen horizontalen Schnitt einer Explosionsdarstellung eines erfindungsgemäßen Gebläsemoduls und
- Figur 6:: eine mehrfach geschnittene perspektivische Ansicht eines erfindungsgemäßen Gebläsemoduls einer Vorrichtung zur Beatmung.

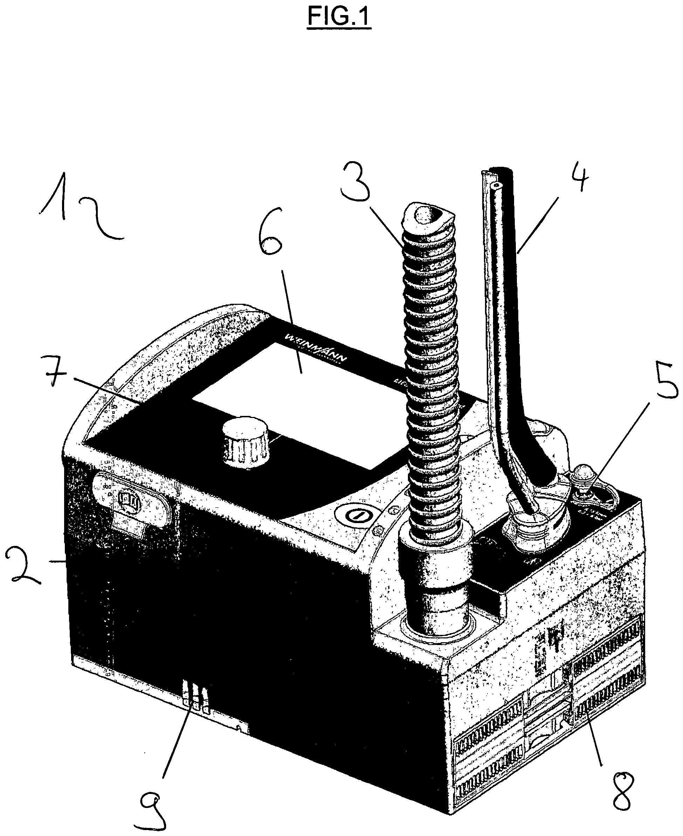

Figur 1 zeigt eine perspektivische Darstellung einer erfindungsgemäßen Vorrichtung zur Beatmung (1). Die Vorrichtung zur Beatmung (1) ist in ein Gehäuse (2) integriert, das in einer vorteilhaften Ausführungsform aus einem stoßfesten Kunststoff gefertigt ist. Im rechten Bereich der Gehäuseoberseite der Vorrichtung zur Beatmung (1) sind Anschlussvorrichtungen angeordnet.

In der dargestellten Ausführungsform weist die Vorrichtung zur Beatmung (1) einen Beatmungsschlauch (3), einen Sauerstoffschlauch und ein Messschlauchsystem (4) sowie einen Druckanschluss (5) für eine Sauerstoffquelle auf.

Als Benutzerschnittstellen weist die Vorrichtung zur Beatmung (1) ein Display (6) und ein als ein Drehknopf ausgebildetes Bedienelement (7) auf.

Auf der rechten Seite des Gehäuses (2) der Vorrichtung zur Beatmung (1) ist in einem unteren Bereich ein Lufteinlass (8) angeordnet und auf der Vorderseite des Gehäuses (2) ist in einem unteren Bereich ein Luftauslass (9) angeordnet.

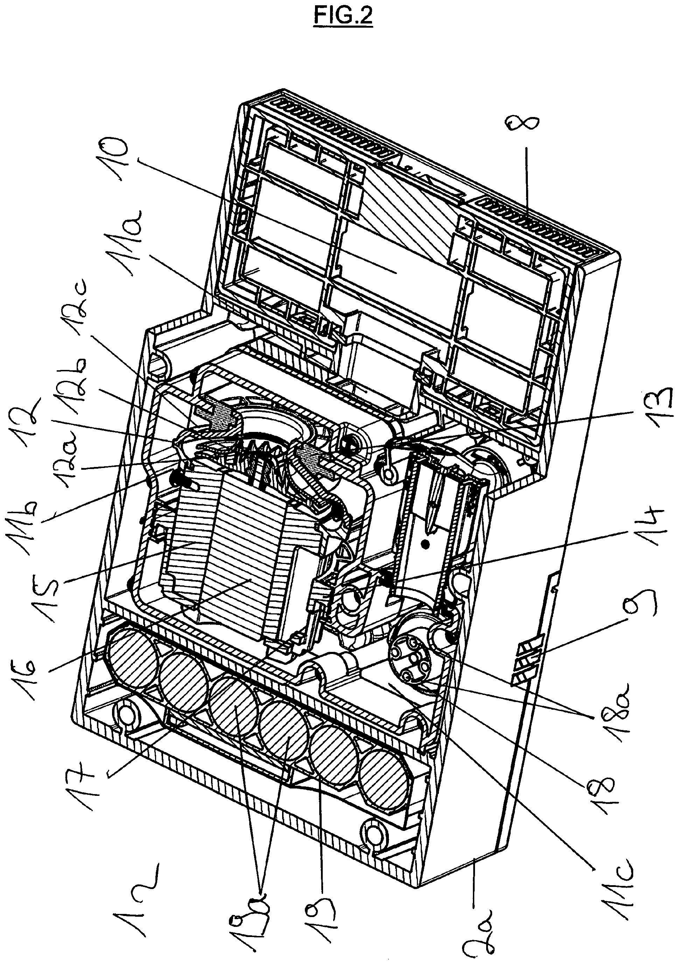

In Figur 2 ist eine mehrfach horizontal geschnittene perspektivische Ansicht einer erfindungsgemäßen Vorrichtung zur Beatmung (1) dargestellt. In ein Gehäuseunterteil (2a) der Vorrichtung zur Beatmung (1) ist auf der rechten Seite der Lufteinlass (8) zu erkennen. Von dem Lufteinlass (8) ist die Umgebungsluft durch einen Filter (10), besonders vorteilhaft ausgebildet als ein Hygienefilter, über einen Ansaugbereich (11) von einem Gebläse (12) ansaugbar.

Das Gebläse (12), ist vorteilhaft als ein Radialgebläse ausgebildet und weist ein Lüfterrad (12a), eine Gebläsewelle (12b) und eine Gebläsekappe (12c) auf, die das Lüfterrad (12a) und die Gebläsewelle (12b) umschließt.

Auf der Seite des Ansaugbereiches (11a) ist das Gebläse (12) in einem ersten Lagerelement (13) gelagert, das erfindungsgemäß zumindest teilweise aus Silikon gefertigt ist. Zudem realisiert das erste Lagerelement (13) eine Dichtung zwischen Ansaugbereich (11a) und einem ersten Überdruckbereich (11b), in den die Luft durch eine Öffnung in der Gebläsekappe (12c) gelangt.

Dieser erste Überdruckbereich (11b) ist durch eine Gehäusestruktur und ein zweites Lagerelement (14) räumlich begrenzt und umschließt den Kühlkörper (15) des Gebläses (12). Mittig in den Kühlkörper (15) ist ein Gebläsemotor (16) integriert, der die Gebläsewelle (12b) antreibt. Das zweite Lagerelement (14) lagert den Kühlkörper (15) und das damit verbundene Gebläse (12) im Gehäuse (2) und ist erfindungsgemäß zumindest teilweise aus Silikon gefertigt, sodass Stöße und Körperschall gedämpft werden. Zusätzlich ist eine Ausströmöffnung (17) derart in dem zweiten Lagerelement (14) angeordnet, dass zusätzlich zu der Lagerung des Gebläses (12) eine Zwangsführung der Luft realisiert ist, sodass die Luft innerhalb des ersten Überdruckbereiches (11b) über die Kühlrippen des Kühlkörpers (15) strömt, bevor diese durch die Ausströmöffnung (17) im zweiten Lagerelement (14) in einen zweiten Überdruckbereich (11c) strömt.

Im Bereich des zweiten Überdruckbereiches (11c) ist eine Kühlblende (18) angeordnet, die zwei zylinderförmige Röhren (18a) aufweist, die über ein Rückschlagventil zum Luftauslass (9) der Vorrichtung zur Beatmung (1) führen.

Im linken Bereich des Gehäuseunterteils (2a) ist ein Energiespeicherelement (19) angeordnet, dass als ein Akkumulator ausgebildet ist und in der dargestellten Ausführungsform sechs Zellen (19a) aufweist.

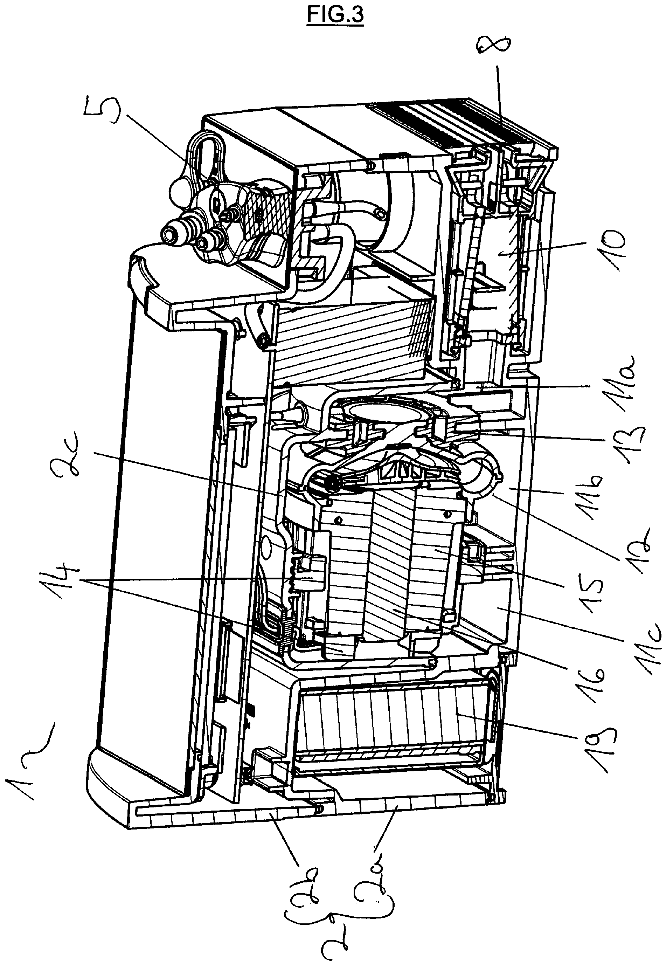

Figur 3 zeigt eine perspektivische Ansicht eines vertikalen Schnittes einer erfindungsgemäßen Vorrichtung zur Beatmung (1). Zu erkennen sind ergänzend zu Figur 3 insbesondere die verschiedenen Druckbereiche (11) im Bereich des Gebläses (12).

Der Ansaugbereich (11a) liegt in dem rechts an das Gebläse (12) angrenzenden Bereich und ist mithilfe des ersten Lagerelementes (13) von dem ersten Überdruckbereich (11b) abgegrenzt und abgedichtet. In den ersten Überdruckbereich (11b) gelangt die Luft durch die Wirkung des Gebläses (12), dass diese aus dem Ansaugbereich (11a) absaugt und in den ersten Überdruckbereich (11b) hineindrückt.

Das Lagerelement (14) trennt den ersten Überdruckbereich (11b) von dem zweiten Überdruckbereich (11c) und realisiert eine Zwangsführung der Luft von dem ersten Überdruckbereich (11b) in den zweiten Überdruckbereich (11c) derart, dass die Luft entlang des Kühlkörpers (15) strömt und ein Wärmeaustausch zwischen Luft und Kühlkörper (15) stattfindet.

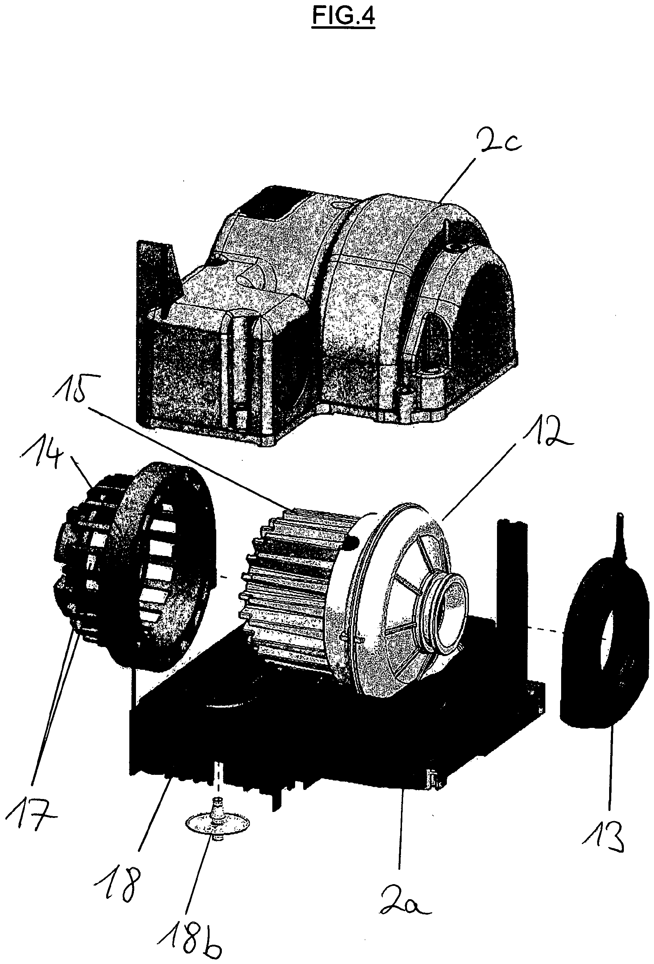

In Figur 4 sind die wesentlichen Elemente zur Veranschaulichung der Leitstrukturen im Bereich des Gebläses (12) perspektivisch dargestellt. Das Gebläse (12) ist mit dem zugeordneten Kühlkörper (15) verbunden. Rechts neben dem Gebläse (12) ist das erste Lagerelement (13) dargestellt, in dem das Gebläse im Bereich der Ansaugöffnung lagerbar ist. An seiner Innenseite weist das erste Lagerelement (13) eine an die Form des Gebläses (12) im Bereich der Ansaugöffnung angepasste Gegenstruktur auf. An seiner Außenseite weist das erste Lagerelement (13) eine radial umlaufende Nut auf, mit der das erste Lagerelement (13) in eine entsprechend federartig ausgebildete Gegenstruktur im Gehäuse (2) der Vorrichtung zur Beatmung (1) einsetzbar ist.

Links neben dem Kühlkörper (15) ist das zweite Lagerelement (14) angeordnet, welches das Gebläse (12) im Bereich des Kühlkörpers (15) lagert. Auf der dem Kühlkörper (15) zugewandten Seite weist das zweite Lagerelement (14) einen verstärkten Wulst auf, der in eine entsprechende Gegenstruktur im Gehäuse (2) einsetzbar ist. Weiterhin ist die Ausströmöffnung (17) des zweiten Lagerelementes (14) als eine Anordnung mehrerer kleinerer Öffnungen realisiert, die in ihrer Größe und Position der Struktur des Kühlkörpers (15) angepasst sind.

Unterhalb des dargestellten Ausschnittes des Gehäuseunterteils (2a) ist im Bereich der Kühlblende (18) eine Rückschlagmembran (18b) gezeigt, die mit den zylinderförmigen Röhren (18a) und der lokalen Struktur des Gehäuseunterteils (2a) das Rückschlagventil realisieren, sodass im Bereich des Luftauslasses (8) keine Luft in die Vorrichtung zur Beatmung (1) eintreten kann.

Innerhalb des Gehäuses (2) der Vorrichtung zur Beatmung (1) ist das Gebläse (2) durch eine Haube (2c) eingefasst.

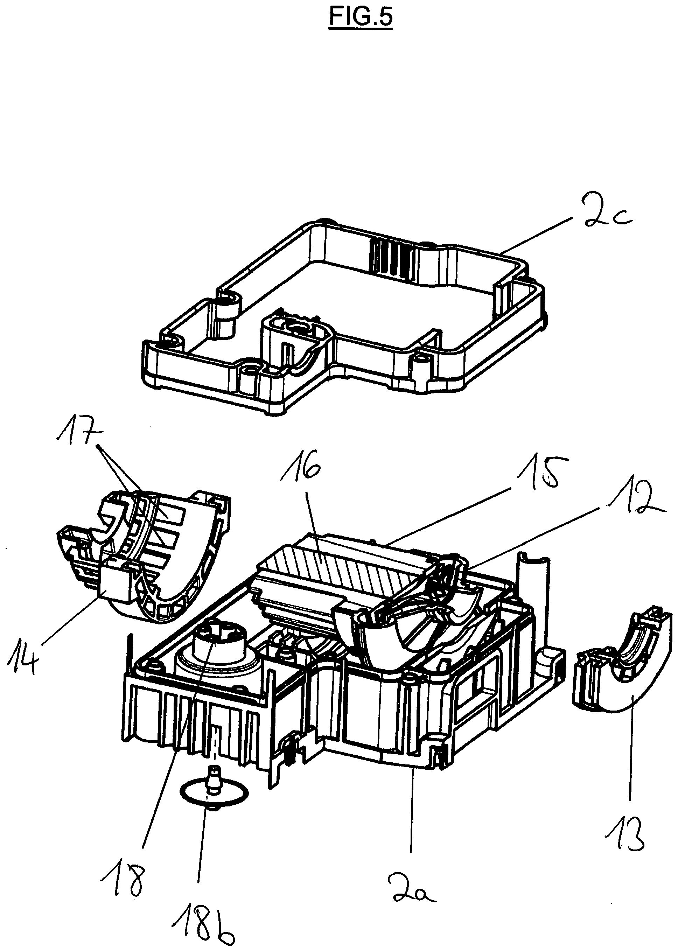

Figur 5 zeigt eine geschnittene Teildarstellung der in Figur 4 dargestellten Elemente der erfindungsgemäßen Vorrichtung zur Beatmung (1).

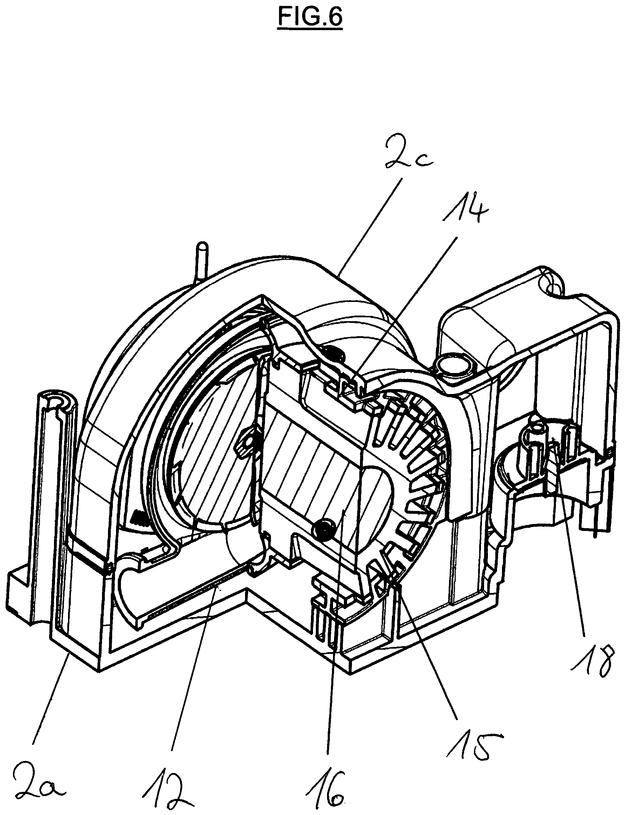

In Figur 6 ist eine mehrfach geschnittene perspektivische Darstellung der in den Figuren 4 und 5 gezeigten Elementen im Bereich des Gebläses (12) einer erfindungsgemäßen Vorrichtung zur Beatmung in einem anderen Blickwinkel dargestellt.

## Patentansprüche

1. Vorrichtung zur Beatmung (1), die mindestens ein Gebläse (12) samt Kühlrippen aufweist, das von einem Gebläsemotor (16) angetrieben wird, und bei der zumindest der Gebläsemotor (16) auf mindestens einem Lagerelement (14) gelagert ist und dass das Lagerelement (14) eine Leitstruktur aufweist, die mithilfe des Gebläses (12) bewegte Kühlluft über die Kühlrippen des Kühlkörpers (15) des Gebläsemotors (16) zwangsführt, wobei das Lagerelement (14) aus Silikon gefertigt ist und wobei das Lagerelement (14) die Leitstruktur für die Kühlluft derart realisiert, dass die Kühlluft angepasst an den Aufbau des Kühlkörpers (15) geleitet wird und dass das Lagerelement (14) über den Kühlkörper (15) stülpbar ist und den Kühlkörper (15) radial umlaufend umschließt.

2. Vorrichtung nach Anspruch 1, **dadurch gekennzeichnet, dass** diese zwei Lagerelemente (13, 14) aufweist, von denen ein erstes Lagerelement (13) das Gebläse (12) auf der Seite des Ansaugbereiches (11a) lagert und das zweite Lagerelement (14) das Gebläse (12) im Bereich des Kühlkörpers (15) lagert.

3. Vorrichtung nach mindestens einem der vorstehenden Ansprüche, **dadurch gekennzeichnet, dass** diese ein Gebläsemodul aufweist, welches zumindest den Kühlkörper (15), den Gebläsemotor (16), ein Lüfterrad (12a), eine Gebläsewelle (12b) und eine Gebläsehaube (12c) aufweist, und dass das Gebläsemodul durch die zwei Lagerelemente (13, 14) aus Silikon in der Vorrichtung zur Beatmung (1) gelagert ist.

4. Vorrichtung nach mindestens einem der vorstehenden Ansprüche, **dadurch gekennzeichnet, dass** mindestens eines der Lagerelemente (13, 14) verschiedene Druckbereiche voneinander trennt und abdichtet.

5. Vorrichtung nach mindestens einem der vorstehenden Ansprüche, **dadurch gekennzeichnet, dass** diese als ein Notfallbeatmungsgerät ausgebildet ist.

6. Vorrichtung nach Anspruch 6, **dadurch gekennzeichnet, dass** diese als ein mobiles Notfallbeatmungsgerät ausgebildet ist.

7. Nicht-therapeutisches Verfahren zum Betrieb der Vorrichtung zur Beatmung gemäß einem der Ansprüche 1 bis 6, bei der das mindestens eine Gebläse (12) samt Kühlrippen verwendet wird, von dem zumindest der Gebläsemotor (16) auf mindestens einem Lagerelement (13, 14) gelagert ist, und bei dem in einem eingeschalteten Zustand der Vorrichtung zur Beatmung (1) permanent Kühlluft abgeblasen wird.

8. Nicht-therapeutisches Verfahren nach Anspruch 7, **dadurch gekennzeichnet, dass** Kühlluft mit einem permanenten Volumenstrom von 25 l/min bis 75 l/min abgeblasen wird.

## Claims

1. Ventilation device (1) having at least one fan (12) and cooling ribs, which fan (12) is driven by a fan motor (16), and in which device at least the fan motor (16) is supported on at least one bearing element (14), and the bearing element (14) has a guide structure by which cooling air, moved with the aid of the fan (12), is positively guided over the cooling ribs of the heat sink (15) of the fan motor (16), wherein the bearing element (14) is made of silicone, and wherein the bearing element (14) implements the guide structure for the cooling air in such a way that the cooling air is guided in a manner adapted to the structure of the heat sink (15), and the bearing element (14) can be slipped over the heat sink (15) and radially peripherally encloses the heat sink (15).

2. Device according to Claim 1, **characterized in that** it has two bearing elements (13, 14), of which a first bearing element (13) supports the fan (12) on the side of the intake region (11a), and the second bearing element (14) supports the fan (12) in the region of the heat sink (15).

3. Device according to at least one of the preceding claims, **characterized in that** it has a fan module which comprises at least the heat sink (15), the fan motor (16), a fan wheel (12a), a fan shaft (12b) and a fan hood (12c), and **in that** the fan module is supported in the ventilation device (1) by the two bearing elements (13, 14) made of silicone.

4. Device according to at least one of the preceding claims, **characterized in that** at least one of the bearing elements (13, 14) separates and seals off various pressure regions from one another.

5. Device according to at least one of the preceding claims, **characterized in that** it is designed as an emergency ventilator.

6. Device according to Claim 6, **characterized in that** it is designed as a mobile emergency ventilator.

7. Non-therapeutic method for operating the ventilation device according to one of Claims 1 to 6, using the at least one fan (12) and cooling ribs, of which at least the fan motor (16) is supported on at least one bearing element (13, 14), and in which method cooling air is permanently blown out in a switched-on state of the ventilation device (1).

8. Non-therapeutic method according to Claim 7, **characterized in that** cooling air is blown out with a permanent volumetric flow of 25 l/min to 75 l/min.

## Revendications

1. Dispositif pour la respiration artificielle (1), qui présente au moins un ventilateur (12) avec des nervures de refroidissement, qui est entraîné par un moteur de ventilateur (16), et dans lequel au moins le moteur de ventilateur (16) est monté sur au moins un élément de palier (14) et en ce que l'élément de palier (14) présente une structure conductrice, qui guide de manière forcée l'air de refroidissement déplacé à l'aide du ventilateur (12) sur les nervures de refroidissement du corps de refroidissement (15) du moteur de ventilateur (16), l'élément de palier (14) étant fabriqué en silicone et l'élément de palier (14) réalisant la structure conductrice pour l'air de refroidissement, de telle sorte que l'air de refroidissement est conduit de manière adaptée à la construction du corps de refroidissement (15) et que l'élément de palier (14) peut être enfoncé sur le corps de refroidissement (15) et entoure le corps de refroidissement (15) radialement sur la périphérie.

2. Dispositif selon la revendication 1, **caractérisé en ce que** celui-ci présente deux éléments de palier (13, 14), parmi lesquels un premier élément de palier (13) supporte le ventilateur (12) du côté de la zone d'aspiration (11a) et le deuxième élément de palier (14) supporte le ventilateur (12) dans la zone du corps de refroidissement (15).

3. Dispositif selon au moins l'une quelconque des revendications précédentes, **caractérisé en ce que** celui-ci présente un module de ventilateur, qui présente au moins le corps de refroidissement (15), le moteur de ventilateur (16), une roue de soufflante (12a), un arbre de ventilateur (12b) et un capot de ventilateur (12c), et **en ce que** le module de ventilateur est monté par les deux éléments de palier (13, 14) en silicone dans le dispositif pour la respiration artificielle (1).

4. Dispositif selon au moins l'une quelconque des revendications précédentes, **caractérisé en ce qu'**au moins l'un des éléments de palier (13, 14) sépare différentes zones de pression les unes des autres et les rend étanches.

5. Dispositif selon au moins l'une quelconque des revendications précédentes, **caractérisé en ce que** celui-ci est conçu comme un respirateur d'urgence.

6. Dispositif selon la revendication 6, **caractérisé en ce que** celui-ci est conçu comme un respirateur d'urgence mobile.

7. Procédé non thérapeutique pour faire fonctionner le dispositif pour la respiration artificielle selon l'une quelconque des revendications 1 à 6, dans lequel l'au moins un ventilateur (12) avec des nervures de refroidissement est utilisé, dont au moins le moteur de ventilateur (16) est monté sur au moins un élément de palier (13, 14), et dans lequel de l'air de refroidissement est évacué par soufflage en permanence dans un état activé du dispositif pour la respiration artificielle (1).

8. Procédé non thérapeutique selon la revendication 7, **caractérisé en ce que** de l'air de refroidissement est évacué par soufflage à un débit volumique permanent de 25 l/min à 75 l/min.
